# EUROPÄISCHE PATENTSCHRIFT

(11) **EP 0 517 789 B1**
(45) Veröffentlichungstag und Bekanntmachung des Hinweises auf die Patenterteilung: **08.06.1994**
(21) Anmeldenummer: 91905556.6
(22) Anmeldetag: 01.03.1991
(51) Int. Cl.: C07K 15/00, A61K 37/02

(54) **INHIBITOR DER PROLIFERATION VON ENDOTHELZELLEN**
INHIBITOR OF PROLIFERATION OF ENDOTHELIAL CELLS
INHIBITEUR DE LA PROLIFERATION DE CELLULES ENDOTHELIALES

(30) Priorität: 02.03.1990 DE 4006609
(43) Veröffentlichungstag der Anmeldung: 16.12.1992
(73) Patentinhaber: Max-Planck-Gesellschaft zur Förderung der Wissenschaften e.V., D-37073 Göttingen (DE)
(72) Erfinder: RISAU, Werner, D-8032 Gräfelfing (DE); DREXLER, Hannes, D-8000 München 70 (DE)
(74) Vertreter: Huber, Bernhard, Dipl.-Chem.
(86) Internationale Anmeldenummer: EP9100388
(87) Internationale Veröffentlichungsnummer: WO9113094

(56) Entgegenhaltungen:
- Chemical Abstracts, Band 113, Nr. 3, 16. Juli 1990, (Columbus, Ohio, US), D. Vilette et al.: "Identification of an endothelial cell growth-inhibitory activity produced by human monocytes", siehe Seiten 487-488, Zusammenfassung 21985s, & Exp. Cell Res. 1990, 188(2), 219-25
- Biological Abstracts, Band 90, 1990, (Philadelphia, PA, US), M. Takigawa et al.: "A clonal human chondrosarcoma cell line produces an nati-angiogenic antitumor factor", seihe Zusammenfassung 53271 & Anticancer Res. 10 (2 Part A), 311-316, 1990
- Chemical Abstracts, Band 113, Nr. 9, 27. August 1990, (Columbus, Ohio, US), M.A. Moses et al.: "Identification of an inhibitor of nevascularization from cartilage", siehe Seite 148, Zusammenfassung 71819m, & Science (Washington, D.C., 1883), 1990, 248(4961), 1408-10

## Beschreibung

Die Bildung neuer Blutkapillaren (Angiogenese) läuft in einer geordneten Folge von Schritten ab: an einem Punkt, an dem ein neuer Gefäßsproß auszuwachsen beginnt (meist im Bereich postkapillärer Venulen), bauen Endothelzellen lokal die Basalmembran ab, wandern in Richtung auf die Quelle eines die Angiogenese stimulierenden Faktors, wachsen und teilen sich, bilden ein Gefäßlumen und treffen auf andere Gefäßsprosse oder bestehende Kapillaren, so daß ein neuer Kapillarabschnitt entsteht, der sich schließlich mit einer neu gebildeten Basalmembran umgibt.

Bei erwachsenen Individuen ist im Normalfall die angiogenetische Aktivität fast vollständig gehemmt. Lediglich bei der Wundheilung und beim weiblichen Geschlecht im Zusammenhang mit dem ovariellen Zyklus laufen intensivere Angiogenese-Vorgänge ab. Im allgemeinen ist jedoch die Umsatzrate der Endothelzellen im Organismus gering. Die vollständige Erneuerung einer bestehenden Endothelzellen-Population dauert Jahre, wobei jedoch ausgeprägte organ- und gewebespezifische Unterschiede vorkommen (Folkman, Medicine 29 (1985), 10-36).

Die strenge Kontrolle, unter der die Angiogenese normalerweise steht, ist beim Wachstum von soliden Tumoren aufgehoben. Für das Wachstum von Tumoren bei einem Durchmesser von über 1 bis 2 mm ist eine starke Angiogenese absolut erforderlich. Avaskuläre Tumoren bleiben nämlich durch die beschränkte Diffusion bei der Zufuhr von Gasen und Nährstoffen und bei der Entfernung von Abfallstoffen auf eine sehr geringe Größe beschränkt. Die mangelnde Fähigkeit von festen Tumoren, in Abwesenheit einer erfolgreichen Induktion der Angiogenese auf eine klinisch signifikante Größe zu wachsen oder Metastasen zu bilden, hat ein großes Interesse bei der Erforschung von Verbindungen hervorgerufen, welche die Angiogenese inhibieren.

Die gewerbliche Anwendbarkeit solcher Inhibitoren liegt bei der Hemmung des Tumorwachstums im allgemeinen, insbesondere bei der Hemmung von auf Endothelzellen basierenden Tumoren wie Kaposi-Sarkom und Hämangiomen. Darüber hinaus ist auch eine therapeutische Anwendbarkeit bei sonstigen Krankheiten gegeben, die auf einem übermäßigen Kapillarwachstum beruhen. Insbesondere zu nennen sind hierbei die diabetische Retinopathie und retrolentale Fibroplasie, beides Augenerkrankungen. Eine weitere Anwendungsmöglichkeit besteht bei der Wundbehandlung, wobei ein Inhibitor der Angiogenese zur Regelung der Wundheilung eingesetzt werden kann, d.h. bei der Verzögerung der Neubildung von Blutgefäßen. Weiterhin besteht auch die Möglichkeit, einen Angiogenese-Inhibitor im Rahmen der Behandlung der rheumatoiden Arthritis einzusetzen. Bei dieser Erkrankung wird eine Vaskularisierung der Knorpel (wie generell bei jeder Entzündung in diesem Bereich) beobachtet, die mit einem Angiogenese-Inhibitor unterdrückt werden kann.

Es wurde aus avaskulären Geweben eine Reihe von die Angiogenese hemmenden Extrakten hergestellt (siehe D'Amore und Braunhut, in Endothelial Cells, Vol. II, herausgegeben von U.S. Ryan, CRC Press, Boca Raton, Florida, 13-37). Auch entzündungshemmende Wirkstoffe unterdrücken die Angiogenese (Robin et al., Arch. Ophthamol. 103 (1985), 284-287; Polverini und Novak, Biochem. Biophys. Res. Comm. 140 (1986), 901-907), wie z.B. Protamin (Taylor und Folkman, Nature 297 (1982), 307-312), angiostatische Steroide (Crum et al., Science 230 (1985), 1375-1378), ein plazentaler RNAse-Inhibitor (Shapiro und Vallee, Proc. Natl. Acad. Sci. USA 84 (1987), 2238-2241) und eine Reihe von Verbindungen, welche die Matrixsynthese und Beständigkeit beeinflussen (siehe z.B. Ingber und Folkman, Lab. Invest. 59 (1988), 44-51). Bei einigen dieser Inhibitoren wurde eine Hemmung des Tumorwachstums oder eine Rückbildung in vivo festgestellt, jedoch nicht bei allen Tumoren. Weiterhin bleibt auch die Toxizität dieser Angiogenese-Inhibitoren ein Problem.

Von Rastinejad et al. (Cell 56 (1989), 345-355) wurde ein Angiogenese-Inhibitor in einem Medium mit Hamsterzellen und Hamster-menschlichen Hybridzellen gefunden, der die Neovaskularisation in vivo unterdrückt. Bei dieser Verbindung handelt es sich offenbar um ein Glykoprotein mit 140 kD Molekulargewicht. Allerdings zeigt dieser Inhibitor nur eine geringe Stabilität, insbesondere ist er nicht hitzestabil.

Aufgabe der vorliegenden Erfindung war es demgemäß, einen stabileren und demnach therapeutisch besser einsetzbaren Angiogenese-Inhibitor bereitzustellen.

Die erfindungsgemäße Aufgabe wird gelöst durch ein Protein, das als Inhibitor der Proliferation von Endothelzellen wirkt, erhältlich aus Babyhamster-Kidney-Zellen (ATCC CCL 10), welcher bei Gelfiltration unter nativen Bedingungen ein Molekulargewicht von ca. 60 bis 100 kD besitzt und eine große Hitzestabilität zeigt.

Bei dem erfindungsgemäßen Inhibitor handelt es sich um ein Protein, da bei Verdau mit Trypsin und Chymotrypsin seine inhibierende Wirkung auf die Endothelzellen-Proliferation vollkommen zerstört wird. Der Inhibitor ist hitzestabil, da er selbst nach 45minütiger Hitzebehandlung bei 60°C noch mindestens 50 % seiner ursprünglichen Aktivität besitzt. Sein Molekulargewicht in nativer Form ergab sich aus Gelfiltrations-Experimenten. Weiterhin zeichnet sich das erfindungsgemäße Protein dadurch aus, daß es nicht an Heparin-Sepharose bindet und sich auf diese Weise von an Heparin bindenden Wachstumsfaktoren für Endothelzellen abtrennen läßt.

Der Nachweis der inhibitorischen Aktivität erfolgte im BAE-Proliferationstest. Dazu wurden Endothelzellen aus der Rinder-Aorta (BAEC = bovine aortic endothelial cells) in einem modifizierten Proliferationstest zur Aktivitätsbestimmung des Inhibitors eingesetzt.

Weiterhin ein Gegenstand der vorliegenden Erfindung ist ein Verfahren zur Gewinnung des erfindungsgemäßen Proteins aus Babyhamster-Kidney-Zellen (ATCC CCL 10). Die Anzucht der Zellen erfolgte in einem Wachstumsmedium unter Zusatz von fötalem Kälberserum, bis ein weitgehend konfluenter Zellrasen erreicht war. Anschließend erfolgt eine Konditionierung des Kulturmediums in serumfreiem Medium, vorzugsweise 2 bis 3 Wochen lang, bis eine gesteigerte Ablösung des Zellrasens von den Gefäßwänden zu beobachten ist. Dann erfolgt eine vollständige Ablösung der Babyhamster-Kidney(BHK)-Zellen durch Proteasebehandlung, vorzugsweise durch Trypsin/EDTA-Behandlung und eine Resuspendierung einzelner Zellen und eine erneute Kultivierung bis zur Konfluenz.

Der erste Schritt der Isolierung des Inhibitors aus BHK konditioniertem Medium ist vorzugsweise eine Ultrafiltration, wobei man eine Ultrafiltrations-Membran mit einer nominellen Ausschlußgrenze von 10 kD verwendet. Dabei wird das Filtrat verworfen und die Proteine im Retentat anschließend einer Ammoniumsulfat-Präzipitation unterworfen.

Dazu wird Ammoniumsulfat bis zu einer ca.75%igen Sättigung zugegeben, wobei das erfindungsgemäße Protein sich im Präzipitat befindet. Das Präzipitat wird durch Zentrifugation gewonnen, resuspendiert und gegen einen geeigneten Puffer dialysiert.

Die Anwesenheit eines Inhibitors der Endothelzellproliferation im von BHK-Zellen konditionierten Medium ist schon nach 10facher Konzentrierung des Mediums im BAETest erkennbar. Die gesamte inhibitorische Aktivität im Medium läßt sich durch die Ammoniumsulfat-Fällung bei 75 % Sättigung präzipitieren.

Gegebenenfalls kann anschließend ein Reinigungsschritt durch Affinitätschromatographie an Heparin-Sepharose erfolgen. Dies dient zur Abtrennung von Angiogenese-Aktivatoren bzw. Inhibitoren, die an Heparin binden. Für die weitere Reinigung werden die nicht an die Säule bindenden Proteine verwendet.

Als nächster Schritt der weiteren Aufreinigung erfolgt vorzugsweise eine Anionenaustausch-Chromatographie an Q-Sepharose. Das erfindungsgemäße Protein bindet an die Anionenaustauscher-Säule und kann mit einem 0 bis 1 mol/l NaCl-Salzgradienten in einem geeigneten Puffer, vorzugsweise 50 mmol/l Tris-HCl, pH 7,5 eluiert werden.

Unter den beschriebenen Bedingungen wird der Inhibitor bei einer NaCl-Konzentration zwischen 0,24 und 0,32 M der Säule eluiert.

Alternativ zu der hier aufgeführten konventionellen Chromatographie mit Q-Sepharose kann dieser Schritt auch durch eine FPLC an einer MonoQ 10/10 Säule (Pharmacia/LKB; Freiburg) durchgeführt werden.

Fraktionen der Anionenaustausch-Chromatographie, welche die inhibitorische Aktivität enthalten, werden zusammengefaßt und durch Ultrafiltration bis zu 40fach konzentriert. Die konzentrierte Probe kann anschließend auf eine Gelfiltrationssäule aufgetragen werden, vorzugsweise TSK HW 55s (Merck, Darmstadt), und das gebundene Protein mit PBS als Elutionsmittel wieder von der Säule entfernt.

Die Gelfiltration an TSK HW55s-Material erbrachte keinen weiteren Reinigungseffekt, jedoch ließ sich dadurch das Molekulargewicht des Inhibitors auf etwa 60 kDa bestimmen. Prallel hierzu durchgeführte Gelfiltrationen unter den gleichen Pufferbedingungen mittels FPLC an einer Superose 12-Säule (Pharmacia/LKB; Freiburg) führten zum gleichen Ergebnis.

Rinderserum-Albumin (BSA), das nach dieser Gelfiltration Fraktionen mit Inhibitor-Aktivität kontaminierte, konnte durch die anschließende Chromatographie an Affi-Gel-Blue entfernt werden, da der größte Teil des Inhibitors nicht von diesem Material gebunden wird. Dieser Schritt läßt sich auch unmittelbar an die Heparin-Sepharose-Chromatographie oder den Ionenaustauscher-Schritt anschließen.

Anschließend erfolgt eine hydrophobe Interaktions-Chromatographie an einer HPLC-Anlage, vorzugsweise an einer HIC-Spherogel CAA-Säule 100x4,6 mm (Beckman Instruments, München). Die Probe wird mit einer hohen Ammoniumsulfat-Konzentration, vorzugsweise 3 mol/l aufgetragen. Die Elution der gebundenen Substanzen erfolgt mit einem linearen umgekehrten Salzgradienten. Um die inhibitorische Aktivität nach diesem Schritt nachweisen zu können, mußten die Fraktionen nach Elution gegen 20 mmol/l Tris-HCl, pH 7,5 dialysiert werden.

Dieser Schritt ist eine weitere wirksame Methode, den Inhibitor anzureichern und gleichzeitig kontaminierende Proteine abzutrennen. Der Inhibitor bindet unter den gewählten Bedingungen an die Matrix und wird 21 bis 23 Minuten nach dem Start des Gradienten von der Säule eluiert.

Das nach dem erfindungsgemäßen Verfahren vorliegende Endprodukt enthält noch geringe Mengen an Fremdproteinen. Durch einen weiteren Reinigungsschritt kann jedoch ein reines Produkt gewonnen werden, bei dem eine Aminosäure-Teilsequenz bestimmt werden kann. Daraus wiederum können DNA-Oligonukleotid-Sonden hergestellt werden und somit über die cDNA oder genomische DNA die gesamte Aminosäuresequenz des Inhibitors vorgenommen werden. Diese Verfahren sind einem Fachmann auf dem Gebiet der Molekularbiologie bekannt.

Als Ausgangsmaterial zur Gewinnung des erfindungsgemäßen Proteins kann neonatales Gewebe verwendet werden, vorzugsweise Babyhamster-Kidney-Zellen (ATCC CCL 10), da das neonatale Gewebe viel höhere Konzentrationen des Inhibitors enthalten dürfte als das Gewebe von Erwachsenen-Organismen, wobei es fraglich ist, ob erwachsene Organismen überhaupt diesen Inhibitor enthalten.

Weiterhin ein Gegenstand der vorliegenden Erfindung ist ein Arzneimittel, welches das erfindungsgemäße Protein als Wirkstoff, sowie übliche Füll-, Träger- oder/und Hilfsstoffe enthält.

Das erfindungsgemäße Protein kann bei der Behandlung aller Krankheiten, bei denen eine gesteigerte Angiogenese stattfindet und die oben bereits erwähnt wurden, verwendet werden. Insbesondere kann das erfindungsgemäße Protein zur Hemmung des Tumorwachstums, wie von Kaposi-Sarcomen oder Hämangiomen, zur Behandlung von Augenerkrankungen, bei welchem gesteigertes Kapillarwachstum auftritt, insbesondere der diabetischen Retinopathie und der retrolentalen Fibroplasie, sowie bei der rheumatoiden Arthritis oder zur Regelung der Wundheilung eingesetzt werden.

Die vorzugsweise verwendete Dosis des erfindungsgemäßen Proteins pro kg Körpergewicht dürfte in der Größenordnung »g bis maximal mg liegen.

Die folgenden Beispiele sollen die Erfindung näher verdeutlichen.

### Beispiel 1

### Zellkultur

Für die Herstellung des konditionierten Mediums wurden Zellen vom Typ BHK-21 (ATCC CCL 10) verwendet (Passage 150-159).

### Anzucht der Zellen

BHK-Zellen wurden zunächst in Costar-Zellkulturflächen mit 75 cm² Wachstumsfläche in DMEM/F12-Medium (1:1; GIBCO, Paisley, Schottland) unter Zusatz von 5 % fötalem Kälberserum (FCS, Boehringer, Mannheim) ausgesät und bis zur Konfluenz der Zellen im Brutschrank bei 37°C, 7% CO₂ und 95% Luftfeuchtigkeit inkubiert.

Nach Erreichen der Konfluenz wurden die Zellen durch Trypsin/EDTA-Behandlung abgelöst, in DMEM/F12-Medium aufgenommen, abzentrifugiert (Häreus, 5 Minuten bei 1000 upm), in DMEM/F12-Medium resuspendiert und in Costar-Zellkulturflaschen mit 165 cm² Wachstumsfläche überführt. Die Inkubation der Zellen bis zum Erreichen der Konfluenz erfolgte unter den gleichen Bedingungen wie oben angegeben.

Die Zellen einer Zellkulturflasche mit 165 cm² Wachstumsfläche wurden daraufhin durch abermalige Trypsin/EDTA-Behandlung abgelöst, in 50 ml DMEM/F12-Medium, 5 % FCS, 10 mM HEPES pH 7,5 resuspendiert und in Zellkultur-Rollerflaschen mit 900 cm² Wachstumsfläche (Costar) überführt. Die Rollerflaschen wurden unbeschichtet oder nach Beschichtung mit Gelatine eingesetzt. Die Anzucht, wie auch die Konditionierung des Kulturmediums (s.u.) der in die Rollerflaschen überführten Zellen erfolgte auf einem Rollersystem (1,5 upm) und einem Vismara-Brutschrank der Fa. Tecnomara bei 37°C ohne Begasung durch Kohlendioxid. Die Konditionierung des Kulturmediums der BHK-Zellen wurde erst begonnen, nachdem die Zellen in den Rollerflaschen einen weitgehend konfluenten Zellrasen gebildet hatten. Bis dahin wurde das Kulturmedium (DMEM/F12, 5 % FCS, 10 mmol/l HEPES pH 7,5) alle 3 Tage ersetzt.

### Konditionierung des Kulturmediums

Die BHK-Zellen einer Rollerflasche wurden in 50 ml serumfreiem Medium (DMEM/F12, 10 mmol/l HEPES pH 7,5) 4 bis 6 Stunden gewaschen, die Waschlösung verworfen und mit 200 ml Medium versetzt. Die Konditionierung des Kulturmediums mit jeweils 200 ml serumfreiem Medium erfolgte für 72 Stunden bei 37°C und 1,5 upm 2 bis 3 Wochen lang, bis eine gesteigerte Ablösung des Zellrasens von den Gefäßwänden zu beobachten war.

Zu diesem Zeitpunkt wurden die BHK-Zellen durch Trypsin/EDTA-Behandlung vollständig von den Gefäßwänden abgelöst und vereinzelt, in 100 ml DMEM/F12, 5 % FCS und 10 mM HEPES resuspendiert und zu gleichen Teilen auf 2 Rollerflaschen verteilt, wo sie wie unter 1.1.1. beschrieben, wieder bis zur Konfluenz kultiviert wurden.

Das konditionierte Medium wurde durch Zentrifugation von abgelösten Zellen befreit und nach Sterilfiltration bei 4°C aufbewahrt.

### Beispiel 2

### Isolierung des Inhibitors aus BHK-konditioniertem Medium

2.1 Konzentrierung des konditionierten Mesiums
   2.1.1. Ultrafiltration
      4 bis 6 Liter des konditionierten Mediums wurden mit Hilfe eines Minitan Ultrafiltrationssystems (Millipore GmbH, Eschborn) auf ein Zehntel des ursprünglichen Volumens konzentriert. Als Ultrafiltrationsmembranen wurden Membranen vom Typ PGLC aus regenerierter Zellulose (Millipore GmbH, Eschborn) mit einer nominellen Ausschlußgrenze von 10 kDa verwendet. Das Filtrat wurde verworfen, die Proteine im Retentat einer Ammoniumsulfat-Präzipitation unterworfen.
   2.1.2. Ammoniumsulfat-Präzipitation
      Für die weitere Konzentrierung des konditionierten Mediums wurden dem Retentat der Ultrafiltration nach Einstellung des pH-Wertes auf 6,5 festes Ammoniumsulfat (232,2 g/l; 75% Sättigung) unter ständigem Rühren in kleinen Portionen zugegeben und die Proteine über Nacht gefällt. Das Präzipitat wurde durch Zentrifugation für 30 Minuten bei 20000 g gewonnen, in 100 bis 150 ml kaltem, destilliertem Wasser gelöst und gegen 3 x 5 l PBS, pH 7,5 dialysiert (nominelle Ausschlußgrenze der Visking-Dialyseschläuche: 12 bis 14 kDa; Fa. Roth). Kleinere Präzipitate, die sich während der Dialyse bildeten wurden vor dem nächsten Reinigungsschritt durch Zentrifugation entfernt.
   2.2 Affinitäts-Chromatographie an Heparin Sepharose
      Heparin-Sepharose (Pharmacia/LKB, Freiburg) in einer 1 x 5 cm Säule wurde mit PBS equilibriert (Flußrate: 40 ml/Stunde) und das gegen PBS dialysierte Material der gleichen Flußrate auf die Säule aufgetragen. Die nicht an die Säule bindenden Proteine wurden für die weitere Reinigung verwendet.
   2.3 Anionenaustausch-Chromatographie an O-Sepharose
      Das von der Heparin-Sepharose nicht gebundene Material wurde mit 50 mmol/l Tris/HCl-Puffer, pH 7,5 auf das dreifache Volumen verdünnt und mit 250 ml/Stunde auf eine mit Q-Sepharose fast flow (Pharmacia/LKB; Freiburg) gepackte Säule (äquilibriert in 50 mmol/l Tris/HCl-Puffer, pH 7,5) gepumpt. Nach dem Waschen der Säule mit dem Auftragspuffer wurden die gebundenen Proteine mit einem linearen Salzgradienten (Gesamtvolumen: 1600 ml) von 0 bis 1 mol/l NaCl in 50 mmol/l Tris/HCl, pH 7,5 eluiert.
   2.4 Gelfiltrations-Chromatographie mit TSK HW55s
      Fraktionen der Anionenaustausch-Chromatographie, die die inhibitorische Aktivität enthielten (siehe Beispiel 3, BAE-Test), wurden zusammengefaßt und in einer Amicon Ultrafiltrationskammer 40fach konzentriert (PM10 Ultrafiltrationsmembran von Amicon).
      Die konzentrierte Probe wurde anschließend auf eine in PBS äquilibrierte TSK HW55s-Säule (Merck, Darmstadt; 1,5 x 70 cm) aufgetragen und die Chromatographie mit PBS als Eluens und einer Flußrate von 20 ml/Stunde durchgeführt.
   2.5 Entfernung von BSA durch Chromatographie an Affi-Gel-Blue
      Aktive Fraktionen aus Schritt 2.4 wurden zusammengefaßt, mit dem gleichen Volumen an 20 mmol/l Natriumphosphat-Puffer, pH 6,8 verdünnt und auf eine mit dem gleichen Puffer äquilibrierten Affi-Gel-Blue-Säule (50 bis 100 mesh; BioRad; 1,5 x 12 cm) mit einer Flußrate von 40 ml/Stunde aufgetragen. Das ungebundene, fast vollständig BSA-freie Material wurde im nächsten Schritt weiterverwendet.
   2.6 Hydrophobe Interaktions-Chromatographie an HIC Spherogel CAA
      Dieser Schritt wurde an einer HPLC-Anlage der Firma Kontron, Eching durchgeführt.
      4 ml Aliquots des ungebundenen Materials aus Schritt 2.5 wurden über Centricon 10 - oder Centricon 30 - Mikrokonzentratoren (Amicon) auf 150 bis 200 »l konzentriert und auf eine HIC-Spherogel CAA-Säule (100 x 4,6 mm; Beckmann Instruments, München) bei einer Ammoniumsulfat-Konzentration von 3 mol/l (50 mmol/l Natriumphosphat pH 6,8 als Puffer) aufgetragen. Die Elution der gebundenen Substanzen erfolgt mit einem linearen, umgekehrten Salzgradienten von 3 mol/l Ammoniumsulfat/50 mmol/l Natriumphosphat, pH 6,8 nach 50 mmol/l Natriumphosphat/1 mmol/l Natriumacetat, pH 6,8 in 35 Minuten.
      Um die inhibitorische Aktivität im BAE-Test (siehe Punkt 1.3) nachweisen zu können, mußten die Fraktionen gegen 20 mmol/l Tris/HCl, pH 7,5 dialysiert werden.

### Beispiel 3

### Nachweis des Inhibitors im BAE-Proliferationstest

Endothelzellen aus der Rinderaorta (BAEC=bovine aortic endothelial cells) wurden in einem modifizierten Proliferationstest zur Identifizierung des Endothelzell-Inhibitors eingesetzt. Pro Vertiefung einer Costar-Mehrfachkulturschale (24 Vertiefungen pro Schale) wurden hierzu 6000 bis 9000 BAEC in DMEM, 5 % FCS ausgesäht und über Nacht bei 37°C/7 % Kohlendioxidgehalt im Brutschrank inkubiert. Am nächsten Tag wurden nicht adherierte Zellen durch einen Mediumwechsel (DMEM/5% FCS und 0,02 ml FGF aus Rinderretinae/ml Medium) entfernt. (Durch den Zusatz von FGF zum Kulturmedium erhalten die Endothelzellen einen mitogenen Stimulus, der ihre Proliferation um das 1,5 bis 3fache gegenüber dem Kontrollwert anregt).

Bis zu 0,04 ml jeder Fraktion, die auf Anwesenheit des Inhibitors getestet werden sollte wurden in jeweils 2 Vertiefungen der Kulturschale (Doppelbestimmung) pipettiert und die Zellenzahlen nach 72 Stunden Inkubation im Brutschrank durch einen Coulter Counter bestimmt.
4. Charakterisierung des Inhibitors
   4.1 Hitzestabilität
      Inhibitorisch aktive Fraktionen wurden für 10 Minuten, bzw. 45 Minuten bei 60°C inkubiert, dann sterilfiltriert und im BAE-Test auf noch vorhandene Aktivität überprüft.
   4.2 Protease-Sensitivität
      0,1 ml einer inhibierenden Fraktion wurden in 20 mM Tris/HCl-Puffer, pH 7,5 für 90 Minuten mit 0,05 mg Trypsin (Sigma) oder Chymotrypsin A4 (Boehringer) bei 37°C inkubiert. Der Verdau wurde anschließend durch Zusatz von Aprotinin (Sigma) gestoppt, die Proben sterilfiltriert und im BAE-Test überprüft.

Die hinterlegte Zellinie ATCC CCL 10 wurde am 10.Juni 1965 bei der American Type Culture Collection, 12301 Parklawn Drive, Rockville, Maryland 20852-1776, USA unter der oben angegebenen Nummer hinterlegt. (siehe Hinterlegungsbescheinigung)

## Patentansprüche (Patentansprüche für folgende(n) Vertragsstaat(en): AT, BE, CH, DE, DK, FR, GB, IT, LI, LU, NL, SE)

1. Protein, das als Inhibitor der Proliferation von Endothelzellen wirkt, erhältlich aus Babyhamster-Kidney-Zellen (ATCC CCL10),
**dadurch gekennzeichnet**,
daß es bei Gelfiltration unter nativen Bedingungen ein Molekulargewicht zwischen 60 und 100 KDa besitzt, nicht an Heparin-Sepharose bindet, Hitzestabilität bei 60°C aufweist und durch Konzentrierung aus konditioniertem Medium von Embryonalzellen durch Ultrafiltration mit einer Ausschlußgrenze von 10 KDa und nachfolgende Ammoniumsulfat-Präzipitation des Retentats und gegebenenfalls Feinreinigung durch Affinitätschromatographie an Heparin-Sepharose, Anionenaustauschchromatographie, Affi-Gelchromatographie sowie hydrophobe Interaktionschromatographie, erhältlich ist.

2. Protein nach Anspruch 1,
**dadurch gekennzeichnet**,
daß es bei 45-minütiger Behandlung bei 60°C noch ungefähr 70 % seiner ursprünglichen Aktivität besitzt.

3. Verfahren zur Anreicherung eines Proteins nach einem der Ansprüche 1 und 2,
**dadurch gekennzeichnet**,
daß man das Protein aus einem konditionierten Medium von Embryonalzellen durch Konzentrierung und Ammoniumsulfat-Präzipitation gewinnt und anschließend eine weitere Reinigung erfolgt.

4. Verfahren nach Anspruch 3,
**dadurch gekennzeichnet**,
daß man als Embryonalzellen Babyhamster-Kidneyzellen (ATCC CCL 10) verwendet.

5. Verfahren nach Anspruch 3 oder 4,
**dadurch gekennzeichnet**,
daß die Konzentrierung des Mediums durch Ultrafiltration mit einer Membran geschieht, die eine nominelle Ausschlußgröße von 10 KDa besitzt, und die Ammoniumsulfat-Präzipitation durch Zugabe von Ammoniumsulfat bis ungefähr 75 % Sättigung geschieht, wobei sich das Protein im Präzipitat befindet.

6. Verfahren nach Anspruch 3, 4 oder 5,
**dadurch gekennzeichnet**,
daß man die weitere Reinigung mit mehreren Chromatographieschritten durchführt, welche eine Anionenaustauscherchromatographie und eine hydrophobe Interaktionschromatographie beinhalten.

7. Verfahren nach einem der Ansprüche 3 bis 6,
**dadurch gekennzeichnet**,
daß man zusätzlich eine Heparin-Affinitätschromatographie, eine Gelfiltrationschromatographie oder/und eine Affi-Gelchromatographie durchführt.

8. Arzneimittel,
**gekennzeichnet durch**
ein Protein nach Anspruch 1 oder 2 als Wirkstoff, gegebenenfalls mit üblichen Träger-, Hilfs- oder/und Füllstoffen.

9. Verwendung eines Proteins nach Anspruch 1 oder 2, zur Herstellung eines Arzneimittels für die Behandlung von Krankheitszuständen, bei denen eine Inhibierung des Kapillarwachstums erforderlich ist.

10. Verwendung eines Proteins nach Anspruch 1 oder 2 zur Herstellung eines Arzneimittels zur Behandlung von Tumoren, rheumatoider Arthritis, diabetischer Retinopathie und retrolentaler Fibroplasie.

11. Verwendung eines Proteins nach Anspruch 1 oder 2 zur Herstellung eines Arzneimittels für die Wundbehandlung, um die Neubildung von Blutgefäßen zu regulieren.

## Patentansprüche (Patentansprüche für folgende(n) Vertragsstaat(en): ES, GR)

1. Verfahren zur Anreicherung eines Proteins, das als Inhibitor der Proliferation von Endothelzellen wirkt, erhältlich aus Babyhamster-Kidney-Zellen (ATCC CCL10),
**dadurch gekennzeichnet,**
daß es bei Gelfiltration unter nativen Bedingungen ein Molekulargewicht zwischen 60 und 100 KDa besitzt, nicht an Heparin-Sepharose bindet, Hitzestabilität bei 60°C aufweist und durch Konzentrierung aus konditioniertem Medium von Embryonalzellen durch Ultrafiltration mit einer Ausschlußgrenze von 10 KDa und nachfolgende Ammoniumsulfat-Präzipitation des Retentats und gegebenenfalls Feinreinigung durch Affinitätschromatographie an Heparin-Sepharose, Anionenaustauschchromatographie, Affi-Gelchromatographie sowie hydrophobe Interaktionschromatographie, erhältlich ist.

2. Verfahren nach Anspruch 1,
**dadurch gekennzeichnet**,
daß man als Embryonalzellen Babyhamster-Kidney-Zellen (ATCC CCL10) verwendet.

3. Verfahren nach Anspruch 1 oder 2,
**dadurch gekennzeichnet**,
daß die Konzentrierung des Mediums durch Ultrafiltration mit einer Membran geschieht, die eine nominelle Ausschlußgröße von 10 KDa besitzt, und die Ammoniumsulfat-Präzipitation durch Zugabe von Ammoniumsulfat bis ungefähr 75 % Sättigung geschieht, wobei sich das Protein im Präzipitat befindet.

4. Verfahren nach Anspruch 1, 2 oder 3,
**dadurch gekennzeichnet**,
daß man die weitere Reinigung mit mehreren Chromatographieschritten durchführt, welche eine Anionenaustauscherchromatographie und eine hydrophobe Interaktionschromatographie beinhalten.

5. Verfahren nach einem der Ansprüche 1 bis 4,
**dadurch gekennzeichnet**,
daß man zusätzlich eine Heparin-Affinitätschromatographie, eine Gelfiltrationschromatographie oder/und eine Affi-Gelchromatographie durchführt.

6. Verwendung eines Proteins, das nach einem der Ansprüche 1 bis 5 erhalten wird, zur Herstellung eines Arzneimittels für die Behandlung von Krankheitszuständen, bei denen eine Inhibierung des Kapillarwachstums erforderlich ist.

7. Verwendung eines Proteins, das nach einem der Ansprüche 1 bis 5 erhalten wird, zur Herstellung eines Arzneimittels zur Behandlung von Tumoren, rheumatoider Arthritis, diabetischer Retinopathie und retrolentaler Fibroplasie.

8. Verwendung eines Proteins, das nach einem der Ansprüche 1 bis 5 erhalten wird, zur Herstellung eines Arzneimittels für die Wundbehandlung, um die Neubildung von Blutgefäßen zu regulieren.

## Revendications (Revendications pour l'(les) Etat(s) contractant(s) suivant(s): AT, BE, CH, DE, DK, FR, GB, IT, LI, LU, NL, SE)

1. Protéine ayant un effet d'inhibiteur de la prolifération des cellules endothéliales, pouvant être obtenue à partir de cellules de rein de hamster nouveau-né (ATCC CCL10), caractérisée en ce qu'elle possède une masse moléculaire, mesurée par filtration sur gel dans des conditions natives, comprise entre 60 et 100 kd, en ce qu'elle ne se lie pas à l'héparine-Sepharose, en ce qu'elle présente une stabilité thermique à 60°C et en ce qu'elle peut être obtenue par concentration à partir d'un milieu conditionné de cellules embryonnaires par ultrafiltration avec un seuil de coupure de 10 kd, puis précipitation du rétentat avec du sulfate d'ammonium et éventuellement purification poussée par chromatographie d'affinité sur héparine-Sepharose, chromatographie par échange d'anions, chromatographie sur Affi-gel et chromatographie par interaction hydrophobe.

2. Protéine selon la revendication 1, caractérisée en ce qu'elle possède encore près de 70 % de son activité d'origine après un traitement de 45 minutes à 60°C.

3. Procédé de concentration d'une protéine selon l'une des revendications 1 et 2, caractérisé en ce que l'on obtient la protéine à partir d'un milieu conditionné de cellules embryonnaires par concentration et précipitation avec du sulfate d'ammonium et que l'on effectue ensuite une purification supplémentaire.

4. Procédé selon la revendication 3, caractérisé en ce que l'on utilise comme cellules embryonnaires des cellules de reins de hamster nouveau-né (ATCC CCL 10).

5. Procédé selon la revendication 3 ou 4, caractérisé en ce que la concentration du milieu s'effectue par ultrafiltration avec une membrane ayant un seuil de coupure nominal de 10 kd, et en ce que la précipitation avec le sulfate d'ammonium s'effectue par addition de sulfate d'ammonium jusqu'à environ 75 % de la saturation, la protéine se trouvant dans le précipité.

6. Procédé selon la revendication 3, 4 ou 5, caractérisé en ce que l'on effectue la purification supplémentaire au moyen de plusieurs étapes de chromatographie, qui comprennent une chromatographie par échange d'anions et une chromatographie par interaction hydrophobe.

7. Procédé selon l'une des revendications 3 à 6, caractérisé en ce que l'on effectue de plus une chromatographie d'affinité sur héparine, une chromatographie de filtration sur gel et/ou une chromatographie sur Affi-gel.

8. Médicaments caractérisés par une protéine selon la revendication 1 ou 2 en tant que substance active, éventuellement avec des supports, adjuvants et charges classiques.

9. Utilisation d'une protéine selon la revendication 1 ou 2 pour la préparation d'un médicament pour le traitement d'états pathologiques dans lesquels une inhibition du développement des vaisseaux capillaires est nécessaire.

10. Utilisation d'une protéine selon la revendication 1 ou 2 pour la préparation d'un médicament pour le traitement de tumeurs, de la polyarthrite rhumatoïde, de la rétinopathie diabétique et de la fibroplasie rétrocristallinienne.

11. Utilisation d'une protéine selon la revendication 1 ou 2 pour la préparation d'un médicament pour le traitement des blessures, destiné à réguler la néoformation des vaisseaux sanguins.

## Revendications (Revendications pour l'(les) Etat(s) contractant(s) suivant(s): GR, ES)

1. Procédé de concentration d'une protéine ayant un effet d'inhibiteur de la prolifération des cellules endothéliales pouvant être obtenue à partir de cellules de rein de hamster nouveau-né (ATCC CCL10), caractérisé en ce que la protéine possède une masse moléculaire, mesurée par filtration sur gel dans des conditions natives, comprise entre 60 et 100 kd, en ce qu'elle ne se lie pas à l'héparine-Sepharose, en ce qu'elle présente une stabilité thermique à 60°C et en ce qu'elle peut être obtenue par concentration à partir d'un milieu conditionné de cellules embryonnaires par ultrafiltration avec un seuil de coupure de 10 kd, puis précipitation du rétentat avec du sulfate d'ammonium et éventuellement purification poussée par chromatographie d'affinité sur héparine-Sepharose, chromatographie par échange d'anions, chromatographie sur Affi-gel et chromatographie par interaction hydrophobe.

2. Procédé selon la revendication 1, caractérisé en ce que l'on utilise comme cellules embryonnaires des cellules de reins de hamster nouveau-né (ATCC CCL 10).

3. Procédé selon la revendication 1 ou 2, caractérisé en ce que la concentration du milieu s'effectue par ultrafiltration avec une membrane ayant un seuil de coupure nominal de 10 kd, et en ce que la précipitation avec le sulfate d'ammonium s'effectue par addition de sulfate d'ammonium jusqu'à environ 75 % de la saturation, la protéine se trouvant dans le précipité.

4. Procédé selon la revendication 1, 2 ou 3, caractérisé en ce que l'on effectue la purification supplémentaire au moyen de plusieurs étapes de chromatographie, qui comprennent une chromatographie par échange d'anions et une chromatographie par interaction hydrophobe.

5. Procédé selon l'une des revendications 1 à 4, caractérisé en ce que l'on effectue de plus une chromatographie d'affinité sur héparine, une chromatographie de filtration sur gel et/ou une chromatographie sur Affi-gel.

6. Utilisation d'une protéine obtenue selon l'une des revendications 1 à 5 pour la préparation d'un médicament pour le traitement d'états pathologiques dans lesquels une inhibition du développement des vaisseaux capillaires est nécessaire.

7. Utilisation d'une protéine obtenue selon l'une des revendications 1 à 5 pour la préparation d'un médicament pour le traitement de tumeurs, de la polyarthrite rhumatoïde, de la rétinopathie diabétique et de la fibroplasie rétrocristallinienne.

8. Utilisation d'une protéine obtenue selon l'une des revendications 1 à 5 pour la préparation d'un médicament pour le traitement des blessures, destiné à réguler la néoformation des vaisseaux sanguins.

## Claims (Claims for the following Contracting State(s): AT, BE, CH, DE, DK, FR, GB, IT, LI, LU, NL, SE)

1. Protein which acts as an inhibitor of the proliferation of endothelial cells which is obtainable from baby hamster kidney cells (ATCC CCL10),
**wherein**
it has a molecular weight between 60 and 100 kDa in gel filtration under native conditions, it does not bind to heparin-Sepharose, it is thermally stable at 60°C and can be obtained by concentration from conditioned medium of embryonic cells by means of ultrafiltration with an exclusion limit of 10 kDa and subsequent ammonium sulphate precipitation of the retentate and, if desired, fine purification by affinity chromatography on heparin-Sepharose, anion-exchange chromatography, Affi gel chromatography as well as hydrophobic interaction chromatography.

2. Protein as claimed in claim 1,
**wherein**
it still has approximately 70 % of its original activity when treated for 45 minutes at 60°C.

3. Process for the isolation of a protein as claimed in one of the claims 1 and 2,
**wherein**
the protein is isolated from a conditioned medium of embryonic cells by concentration and ammonium sulphate precipitation and subsequently a further purification is carried out.

4. Process as claimed in claim 3,
**wherein**
baby hamster kidney cells (ATCC CCL 10) are used as embryonic cells.

5. Process as claimed in claim 3 or 4,
**wherein**
the medium is concentrated by ultrafiltration with a membrane which has a nominal exclusion size of 10 kDa and the ammonium sulphate precipitation is carried out by addition of ammonium sulphate up to approximately 75 % saturation whereby the protein is located in the precipitate.

6. Process as claimed in claim 3, 4 or 5,
**wherein**
the further purification is carried out using several chromatographic steps which include an anion-exchange chromatography and a hydrophobic interaction chromatography.

7. Process as claimed in one of the claims 3 to 6,
**wherein**
in addition a heparin affinity chromatography, a gel filtration chromatography or/and an Affi-gel chromatography is carried out.

8. Pharmaceutical agent
**characterized by**
a protein as claimed in claim 1 or 2 as the active substance, if desired, together with the usual carrier, auxiliary or/and filling agents.

9. Use of a protein as claimed in claim 1 or 2 to produce a pharmaceutical agent for treating disease states in which an inhibition of capillary growth is necessary.

10. Use of a protein as claimed in claim 1 or 2 to produce a pharmaceutical agent for treating tumours, rheumatoid arthritis, diabetic retinopathy and retrolental fibroplasia.

11. Use of a protein as claimed in claim 1 or 2 to produce a pharmaceutical agent for the treatment of wounds in order to regulate the regeneration of blood vessels.

## Claims (Claims for the following Contracting State(s): GR, ES)

1. Process for the isolation of a protein which acts as an inhibitor of the proliferation of endothelial cells obtainable from baby hamster kidney cells (ATCC CCL10),
**wherein**
it has a molecular weight between 60 and 100 kDa in gel filtration under native conditions, it does not bind to heparin-Sepharose, it is thermally stable at 60°C and can be obtained by concentration from conditioned medium of embryonic cells by means of ultrafiltration with an exclusion limit of 10 kDa and subsequent ammonium sulphate precipitation of the retentate and, if desired, fine purification by affinity chromatography on heparin-Sepharose, anion-exchange chromatography, Affi gel chromatography as well as hydrophobic interaction chromatography.

2. Process as claimed in claim 1,
**wherein**
baby hamster kidney cells (ATCC CCL 10) are used as embryonic cells.

3. Process as claimed in claim 1 or 2,
**wherein**
the medium is concentrated by ultrafiltration with a membrane which has a nominal exclusion size of 10 kDa and the ammonium sulphate precipitation is carried out by addition of ammonium sulphate up to approximately 75 % saturation whereby the protein is located in the precipitate.

4. Process as claimed in claim 1, 2 or 3,
**wherein**
the further purification is carried out using several chromatographic steps which include an anion-exchange chromatography and a hydrophobic interaction chromatography.

5. Process as claimed in one of the claims 1 to 4,
**wherein**
in addition a heparin affinity chromatography, a gel filtration chromatography or/and an Affi-gel chromatography is carried out.

6. Use of a protein which is obtained according to one of the claims 1 to 5 to produce a pharmaceutical agent for treating disease states in which an inhibition of capillary growth is necessary.

7. Use of a protein which is obtained according to one of the claims 1 to 5 to produce a pharmaceutical agent for treating tumours, rheumatoid arthritis, diabetic retinopathy and retrolental fibroplasia.

8. Use of a protein which is obtained according to one of the claims 1 to 5 to produce a pharmaceutical agent for the treatment of wounds in order to regulate the regeneration of blood vessels.
